# EUROPÄISCHE PATENTANMELDUNG

(11) **EP 1 297 818 A2**
(43) Veröffentlichungstag der Anmeldung: **02.04.2003**
(21) Anmeldenummer: 02021773.3
(22) Anmeldetag: 26.09.2002
(51) Int. Cl.: A61K 7/13

(54) **Haarfärbemittel**

(30) Priorität: 26.09.2001 DE 10147564; 26.09.2001 DE 10147577; 26.09.2001 DE 10147576; 26.09.2001 DE 10147580
(71) Anmelder: KPSS-Kao Professional Salon Services GmbH, 64297 Darmstadt (DE)
(72) Erfinder: Lorenz, Heribert, 64401 Gross-Bieberau (DE)

(57) **Zusammenfassung**

Die Haarfärbung wird wesentlich intensiviert, wenn man einem Mittel zum Färben von menschlichen Haaren, enthaltend mindestens ein Oxidationsfarbstoffvorprodukt, ausgewählt aus 4-Aminophenol und dessen Derivaten der allgemeinen Formel (I) 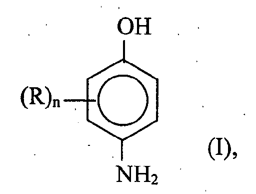 worin R eine C₁-C₃-Alkylgruppe, eine Hydroxy-C₁-C₃-alkylgruppe oder ein Halogenatom, insbesondere Cl, und n eine Zahl von 0 bis 2 bedeuten, oder 2-Aminophenol und/oder 1,4-Diaminobenzol und substituierten p-Phenylendiaminen und/oder Pyrazol und seinen Derivaten und/oder substituierten Triazolen und/oder Aminopyridinen bzw. deren wasserlöslichen Salzen, und vorzugsweise mindestens eine Kupplersubstanz in wäßriger Grundlage 0,0001 bis 5 Gew.-% Dihydroxyaceton und/oder Alloxan und/oder Methylglyoxal und/oder Kaliumjodid, berechnet auf die Gesamt-zusammensetzung des Mittels, zusetzt.

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen wird den farbtechnischen Wünschen der Anwender zwar weitgehend gerecht, es gibt jedoch immer noch Farbnuancen, die dadurch nicht voll erreicht bzw. noch intensiviert werden können.
Es wurde auch bereits vorgeschlagen, diese Lücke durch Verwendung alternativer Entwicklersubstanzen zu schließen. Dies ist in beschränktem Umfang möglich durch den Einsatz von 2-(2,5-Diaminophenyl)ethanol (vgl. EP-A 7537 und EP-B 400 330); jedoch müssen dann Abstriche in der Farbintensität anderer Nuancen hingenommen werden.
Eine weitere befriedigende Lösung dieses Problems wird auch durch den in der EP-A 615 743 beschriebenen Einsatz von 2-(2'-Hydroxyethylamino)-5-aminotoluol bzw. dessen wasserlöslichen Salzen als Entwicklersubstanzen in Haarfärbemitteln erreicht.
Auch die einschlägige Verwendung von 4-Aminophenolen und 2-Aminophenol ist bereits vorgeschlagen worden.
Selbst dadurch bleiben jedoch noch farbtechnische Wünsche offen.

Die Erfindung geht daher von der Aufgabenstellung aus, ein Haarfärbemittel zu schaffen, das zur Herstellung einer großen Anzahl von Farbtönen geeignet ist und vor allem eine besonders intensive glänzende Färbung bewirkt.

Diese Aufgabe wird dadurch gelöst, daß ein solches Haarfärbemittel mindestens ein mit Peroxid reagierendes Oxidatiorisfarbstoffvorprodukt enthält, das ausgewählt ist aus 4-Aminophenol und dessen Derivaten der allgemeinen Formel (I) 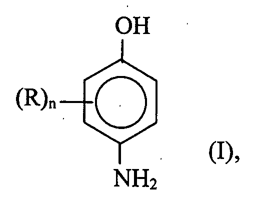 worin R eine C₁-C₃-Alkylgruppe, eine Hydroxy-C₁-C₃-alkylgruppe oder ein Halogenatom, insbesondere Cl, und n eine Zahl von 0 bis 2 bedeuten, oder 2-Aminophenol, 4-Amino-3-methylphenol, 2-Chlor-4-aminophenol, 2,6-Dichlor-4-aminophenol, 2,4-Diaminophenol, 2,6-Dibrom-4-aminophenol und/oder 1,4-Diaminobenzol und substituierten p-Phenylendiaminen, insbesondere 2,5-Diaminotoluol, 2-n-Propyl- bzw. 2-Ethyl-p-phenylendiamin, 2,6 Dimethyl-p-phenylendiamin, 2-(2,5-Diaminophenyl)ethanol, 1-Amino-4-bis-(2'-hydroxyethyl)aminobenzol, 2-(2-Hydroxyethylamino)-5-aminotoluol, 4,4'-Diaminodiphenylamin, 4-Aminodiphenylamin, 2-Amino-5-N,N-diethylaminotoluol, 4-Amino-N-ethyl-N-isopropylanilin, 2-Chlor-p-phenylendiamin, 1-β-Hydroxyethyl-2,5-diamino-4-chlorbenzol, 1-β-Hydroxyethyl-2,5-diamino-4-methylbenzol, 2-Methoxy-p-phenylendiamin, N,N-Di-ethyl-p-phenylendiamin, 1-Amino-4-β-methoxyethylaminobenzol, 1-Dimethylamino-4-amino-benzol, 1-Hydroxy-2,5-diamino-4-methylbenzol, 1-Hydroxymethyl-2,5-diaminobenzol, 1,3-Dimethyl-2,5-diaminobenzol, 1,4-Diaminoisopropylbenzol und/oder 1-Amino-4-β-hydroxypropylaminobenzol bzw. deren wasserlöslichen Salzen und/oder Pyrazol, insbesondere 1-Hydroxyethyl-4,5-diaminopyrazol, 3,4-Diamino-5-hydroxypyrazol, 3,5-Diaminopyrazol, 3,5-Diaminopyrazol-1-carboxamid, 3-Amino-5-hydroxypyrazol, 1-Phenyl-2-methylpyrazol, 1-Phenyl-3-methylpyrazol-5-on, 3,5-Dimethylpyrazol, 3,5-Dimethylpyrazol-1-methanol und/oder 3,5-Diamino-1,2,4-triazol bzw. deren wasserlösliche Salze und/oder Aminopyridine, insbesondere 2,5-Diaminopyridin, 2,3-Diaminopyridin, 2,6-Diaminopyridin, 3-Amino-2-methylamino-6-methoxypyridin, 2-Dimethyl-5-aminopyridin, 2-Dimethylaminoethyl-3-hydroxypyridin, 2-Amino-4,6-dimethylpyridin, 2-Amino-3-hydroxypyridin, 3-Amino-2(β-hydroxyethylamino)-6-methoxypyridin, 2,6-Dimethylamino-5-aminopyridin, 2-Di(hydroxyethyl)amino-5-aminopyridin, 2-Hydroxyethylamino-5-aminopyridin und/oder deren wasserlösliche Salze, in wässriger Grundlage vorliegt, gekennzeichnet durch einen Gehalt an 0,0001 bis 5 Gew.-% Dihydroxyaceton und/oder Alloxan und/oder Methylglyoxal und/oder Kaliumjodid, berechnet auf die Gesamtzusammensetzung.

Vorzugsweise enthält das erfindungsgemäße Mittel mindestens eine Kupplersubstanz, die ausgewählt sein kann aus Resorcin, 2-Methylresorcin, 4-Chlorresorcin, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethyaminophenol, 2,6-Dihydroxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1,3-Diaminobenzol, 1-Amino-3-(2'-hy-droxyethylamino)benzol, 1-Amino-3-[bis(2'-hydroxyethyl)amino]benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 4-Hydroxy-1,2-methyldioxybenzol, 2,4-Diamino-3-chlorphenol, 5-Amino-2-methoxyphenol und/oder 1-Methoxy-2-amino-4-(2'hydroxyethylamino)benzol bzw. deren wasserlöslichen Salzen.
Damit soll jedoch der Zusatz weiterer Entwickler- und Kupplersubstanzen keineswegs ausgeschlossen sein.
Bei Anwendung dieser Zusammensetzungen auf Basis einer üblichen Grundlage werden nach der Oxidation mit Peroxid sehr ausdrucksvolle, intensive, dauerhafte Haarfärbungen erhalten, die durch Zusatz entsprechender weiterer Entwickler- und Kupplersubstanzen noch zu anderen Farbnuancen variiert werden können.

Auch die zusätzliche Mitverwendung weiterer, an sich bekannter Entwicklersubstanzen ist möglich. Hierbei sind insbesondere noch substituierte p-Phenylendiamine wie 2,5-Diaminotoluol, 2-n-Propyl- bzw. 2-Ethyl-p-phenylendiamin, 2,6-Dimethyl-p-phenylendiamin, 2-(2,5-Diaminophenyl)ethanol, 1-Amino-4-bis-(2'-hydroxyethyl)aminobenzol, 2-(2-Hydroxyethylamino)-5-aminotoluol, 4,4'-Diaminodiphenylamin, 4-Aminodiphenylamin, 2-Amino-5-N,N-diethylamlnotoluol, 4-Amino-N-ethyl-N-isopropylanilin, 2-Chlor-p-phenylendiamin, 1-β-Hydroxyethyl-2,5-diamino-4-chlorbenzol, 1-β-Hydroxyethyl-2,5-diamino-4-methylbenzol, 2-Methoxy-p-phenylendiamin, N,N-Diethyl-p-phenylendiamin, 1-Amino-4-β-methoxyethyl-aminobenzol, 1-Dimethylamino-4-aminobenzol, 1-Hydroxy-2,5-diamino-4-methylbenzol, 1-Hydroxymethyl-2,5-diaminobenzol, 1,3-Dimethyl-2,5-diaminobenzol, 1,4-Diaminoisopropylbenzol, 1-Amino-4-β-hydroxypropylaminobenzol, Pyrazol- bzw. Triazolderivate wie 1-Hydroxyethyl-4,5-diaminopyrazol, 3,4-Diamino-5-hydroxypyrazol, 3,5-Diaminopyrazol, 3,5-Diaminopyrazol-1-carboxamid, 3-Amino-5-hydroxypyrazol, 1-Phenyl-2-methylpyrazol, 1-Phenyl-3-methylpyrazol-5-on, 3,5-Dimethylpyrazol, 3,5-Dimethylpyrazol-1-methanol, 3,5-Diamino-1,2,4-triazol, 2-Aminophenol, 4-Aminophenol und dessen Derivate wie 4-Amino-3-methylphenol, 2-Chlor-4-aminophenol, 2,6-Dichlor-4-aminophenol, 2,4-Diaminophenol, 2,6-Dibrom-4-aminophenol, Tetraaminopyrimidine, Triaminohydroxypyrimidine, Diaminomono- und -dihydroxypyrimidine, Aminotriazine, 5-Aminosalicylsäure und/oder 1,2,4-Triaminobenzol und deren wasserlösliche Salze zu erwähnen.
Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3% Gew.-% der Gesamtzusammensetzung des Haarfärbemittels (ohne Oxidationsmittel), wobei sich die Angaben jeweils auf den Anteil an freier Base beziehen.
Das bevorzugte Gewichtsverhältnis der genannten Entwicklersubstanzen zu den weiteren Entwickler- und Kupplersubstanzen liegt dabei zwischen etwa 1 : 8 bis 8 : 1, vorzugsweise etwa 1 : 5 bis 5 : 1, insbesondere 1 : 2 bis 2 : 1.
Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,01 bis 5,0 %, vorzugsweise 0,05 bis 4 %, insbesondere 0,1 bis 3 Gew.-% der Gesamtzusammensetzung (ohne Oxidationsmittel), wobei sich die Angaben jeweils auf den Anteil an freier Base beziehen.
Der Anteil an Dihydroxyaceton und/oder Alloxan und/oder Methylglyoxal und/oder Kaliumjodid liegt vorzugsweise bei etwa 0,0001 bis 5, insbesondere bei etwa 0,01 bis 1 Gew.-% des Färbemittels (ohne Oxidationsmittelzusammensetzung).
Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.
Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 12-prozentiger Konzentration.
Es können jedoch auch andere Peroxide wie Harnstoffperoxid und Melaminperoxid eingesetzt werden.
Alternativ zur Peroxidoxidation kann auch eine Luftoxidation vorgenommen werden, beispielsweise, indem eine ein Oxidationsfarbstoffvorprodukt enthaltende Zusammensetzung als Aerosolschaum auf das Haar aufgebracht und dort für etwa 15 bis 30 Minuten einwirken gelassen wird.
Der pH-Wert des applikationsfertigen Haarfärbemittels, d. h. nach Vermischung mit Peroxid, kann sowohl im schwach sauren, z.B.. einem Bereich von 5,5 bis 6,9, im neutralen als auch im alkalischen Bereich, d. h. zwischen pH 7,1 und 11 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrölysat | 0,5 |
| Ascorbinsäure | 0,2 |
| Parfum | 0,4 |
| Ammoniak, 25%ig | 1,0 |
| Ammoniumchlorid | 0,5 |
| Panthenol | 0,8 |
| Kaliumjodid | 0,0001 bis 0,50 |
| Dihydroxyaceton und/oder Alloxan und/oder | 0,5 bis 5,0 |
| Methylglyoxal | |
| Wasser | ad 100,00 |

Die erfindungsgemäßen Oxidationsfarbstoff-Kombinationen und Dihydroxyaceton wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung (pH-Wert der Mischung: 9,8) und zwanzigminütiger Einwirkung bei Zimmertemperatur, folgendem Auswaschen und Trocknen.

Es wurden die folgenden Färbungen erzielt:

### 1.

| | 1 | 2 | 3 | 4 | 5 | 6 |
|---|---|---|---|---|---|---|
| p-Aminophenol | 0,25 | | | | | |
| 2-Chlor-4-aminophenol | | | 0,32 | | | |
| 2,6-Dichlor-4-aminophenol | | 0,40 | | | | |
| Hydroxyethyl-p-phenylendiaminsulfat | | | | | 0,19 | |
| 4-Amino-3-methylphenol | | | | 0,27 | 0,85 | |
| 2-Aminophenol | | | | | | 0,25 |
| Resorcin | | | | | 0,20 | |
| m-Aminophenol | 0,25 | | | | | |
| 4-Amino-2-hydroxytoluol | | | | | 0,38 | |
| 2-Amino-4-hydroxyethylaminoanisolsulfat | | | | 0,62 | 0,18 | |
| 2-Amino-3-hydroxypyridin | | | 0,25 | | | 0,25 |
| 1-Naphthol | | 0,32 | | | | |
| Dihydroxyaceton | | | 0,50 | | | |
| Alloxan | | 0,50 | | 0,25 | 0,50 | |
| Methylglyoxal | 0,50 | | | 0,25 | | 0,50 |
| Kaliumjodid | 0,002 | 0,002 | 0,002 | | | |

| **Färbeergebnisse:** | **Ohne zusätzliche Wirkstoffe** | **Mit Wirkstoffkombination** |
|---|---|---|
| 1 | Hellbraun | Intensives Nußbraun |
| 2 | Graublau | Tiefblau |
| 3 | Hellkupfergold | Intensives Kupfergold |
| 4 | Graurubin | Intensives Dunkelmagenta |
| 5 | Mahagoni | Intensives Dunkelmahagoni |
| 6 | Beige | Intensives Braunbeige |

### 1a.

In die beschriebene Grundlage wurden die folgenden Oxidationsfarbstoffmischungen jeweils mit und ohne Wirkstoffe eingebracht und der pH-Wert so eingestellt, daß beim Vermischen mit 2%-iger wäßriger H₂O₂-Lösung im Gewichtsverhältnis 1:1 ein pH-Wert der applikationsfertigen Mischung von 6,8 erreicht wurde.
Die Mischungen wurden wiederum jeweils auf Woll-Läppchen und Strähnen aus gebleichtem Menschenhaar aufgebracht, nach 15-minütiger Einwirkung ausgewaschen und getrocknet und die Färbung bewertet.
Es wurde folgendes Ergebnis erzielt:

| Nr. | 7 | 7a | 8 | 8a |
|---|---|---|---|---|
| 2-Chlor-4-aminophenol | 0,32 | 0,32 | 0,32 | 0,32 |
| m-Phenylendiamin | 0,25 | 0,25 | ― | ― |
| 3-Aminophenol | ― | ― | 0,25 | 0,25 |
| Alloxan | 0,50 | ― | ― | ― |
| Methylglyoxal | ― | ― | 0,50 | ― |
| Kaliumjodid | 0,002 | ― | 0,002 | ― |
| Färbung | Grau | | Beige | |

Die erfindungsgemäßen Zusammensetzungen Nr. 7 und 8 erzielten glänzende, intensive Ausfärbungen, die denjenigen der Zusammensetzungen 7a und 8a deutlich überlegen waren.

### 2.

| | 9 | 10 | 11 | 12 | 13 | 14 | 15 | 16 | 17 | 18 | 19 |
|---|---|---|---|---|---|---|---|---|---|---|---|
| p-Phenylendiamin | 0,24% | | | | | | | | | | |
| p-Toluylendiaminsulfat | | 1,01 | 0,54 | 0,88 | | | | | | | |
| Hydroxyethyl-p-phenylendiaminsulfat | | | | | 0,56 | | | | | | |
| N,N-Bis-Hydroxyethyl-p-phenylendiaminsulfat | | | | | | 0,66 | | | | | |
| 2-(2'-Hydroxyethylamino)-5-aminotoluolsulfat | | | | | | | 0,71 | | | | |
| 2-Amino-5-diethylaminotoluolhydrochlorid | | | | | | | | 0,48 | | | |
| 4-Amino-N-ethyl-N-iso-propylanilin | | | | | | | | | 0,48 | 0,48 | |
| 1-β-Hydroxyethyl-2,5-di-amino-4-methylbenzol | | | | | | | | | | | 0,60 |
| Resorcin | | | 0,21 | 0,03 | | | | 0,25 | | 0,25 | |
| m-Aminophenol | | | 0,23 | 0,11 | | | 0,13 | | | | |
| m-Phenylendiamin | | | | | | | | | 0,24 | | |
| 4-Amino-2-hydroxytoluol | | 0,55 | 0,04 | | | | | | | | 0,30 |
| 2-Amino-4-hydroxyethylaminoanisolsulfat | | | | | | 0,63 | | | | | |
| 2-Amino-3-hydroxypyridin | 0,25% | | | 0,2 | 0,25 | | | | | | |
| 1-Naphthol | | | | 0,16 | | | 0,16 | | | | |
| Alloxan | 0,50 | | | 0,40 | | | 0,25 | | 025 | 0,50 | |
| Methylglyoxal | | 0,50 | | | 0,45 | | 0,25 | 0,25 | | | 0,50 |
| Dihydroxyaceton | | | 0,50 | | | 0,40 | | 0,25 | 0,25 | | |
| Kaliumjodid | 0,002 | 0,002 | 0,002 | 0,005 | 0,003 | 0,005 | | | | | |

| **Färbeergebnisse:** | **Ohne zusätzliche Wirkstoffe** | **Mit Wirkstoffkombination** |
|---|---|---|
| 9 | Rotbraun | Intensives Rotbraun |
| 10 | Violett | Tiefviolett |
| 11 | Mittelblond | Intensives Mittelblond |
| 12 | Mahagoni | Intensives Mahagoni |
| 13 | Rotbraun | Intensives Rotbraun |
| 14 | Azurblau | Dunkles Azurblau |
| 15 | Marineblau | Dunkles Marineblau |
| 16 | Mattrot | Graubraun |
| 17 | Türkis | Dunkeltürkis |
| 18 | Mattrot | Graubraun |
| 19 | Violett | Tiefviolett |

### 2a.

In die beschriebene Grundlage wurden die folgenden Oxidationsfarbstoffmischungen jeweils mit und ohne 0,5 Gew.-% Dihydroxyaceton und 0,005 Gew.-% Kaliumjodid eingebracht und der pH-Wert so eingestellt, daß beim Vermischen mit 2%-iger wäßriger H₂O₂-Lösung im Gewichtsverhältnis 1:1 ein pH-Wert der applikationsfertigen Mischung von 6,8 erreicht wurde.
Die Mischungen wurden wiederum jeweils auf Woll-Läppchen und Strähnen aus gebleichtem Menschenhaar aufgebracht, nach 15-minütiger Einwirkung ausgewaschen und getrocknet und die Färbung bewertet.
Es wurde folgendes Ergebnis erzielt:

| Nr. | 20 | 20a | 21 | 21a | 22 | 22a |
|---|---|---|---|---|---|---|
| p-Toluylendiaminsulfat | 1,40 | 1,40 | 0,45 | 0,45 | 1,17 | 1,17 |
| Resorcin | 0,60 | 0,60 | 0,10 | 0,10 | ― | ― |
| 3-Aminophenol | 0,10 | 0,10 | 0,07 | 0,07 | ― | ― |
| 2-Amino-4-hydroxyethylaminoanisolsulfat | 0,02 | 0,02 | ― | ― | ― | ― |
| 4-Amino-2-hydroxytoluol | ― | ― | 0,01 | 0,01 | 0,60 | 0,60 |
| 1-Naphthol | ― | ― | ― | ― | 0,15 | 0,15 |
| 2,5,6-Triamino-4-hydroxypyrimidinsulfat | ― | ― | 0,01 | 0,01 | ― | ― |
| Dihydroxyaceton | 0,50 | ― | 0,50 | ― | 0,50 | ― |
| Kaliumjodid | 0,005 | ― | 0,005 | ― | 0,005 | ― |
| Färbung | Hellbraun | | Mittelblond | | Violett | |

Die erfindungsgemäßen Zusammensetzungen Nr. 20, 21 und 22 erzielten sämtlich glänzende, intensive Ausfärbungen, die denjenigen der Zusammensetzungen 20a, 21a und 22a deutlich überlegen waren.

### 2b.

Es wurde ein Farbschaumaerosol der folgenden Zusammensetzung hergestellt:

| | |
|---|---|
| p-Toluylendiaminsulfat | 0,60 (Gew.-%) |
| N,N-Bis(hydroxyethyl)-p-phenylendiaminsulfat | 0,20 |
| Resorcin | 0,20 |
| 3-Aminophenol | 0,15 |
| 1-Naphthol | 0,05 |
| Natriumsulfit | 0,10 |
| Ascorbinsäure | 0,10 |
| Cocamide MEA | 0,15 |
| Stearamide MEA | 0,13 |
| Parfum | 0,15 |
| Hydroxyethylcellulose | 0,30 |
| Ammoniumchlorid | 0,25 |
| Cocoamidopropylbetain | 2,00 |
| Oleth-5 | 1,00 |
| Cetearylalkohol | 0,70 |
| Konservierungsmittel | 0,10 |
| EDTA | 0,10 |
| Ethanolamin | ad pH 8,5 |
| Wasser | ad 100,00 |

100 Gewichtsteile dieser Zusammensetzung wurden mit 6 Gewichtsteilen eines handelsüblichen Treibmittelgemisches aus Propan/Butan in eine mit einem Schaumventil versehenen Aerosoldose abgefüllt.

Einem Teil der Zusammensetzung wurden 0,7 Gew.-% Dihydroxyaceton und 0,25 Gew.-% Alloxan zugesetzt.
In einem Halbseitenversuch wurde auf die nassen Haare einer Kopfhälfte die Zusammensetzung ohne, auf die andere Kopfhälfte die Zusammensetzung mit Dihydroxyaceton und Alloxan gleichmäßig aufgebracht.
Nach 15-minütiger Einwirkung wurde gespült, getrocknet und beide Haarhälften miteinander verglichen.
Die mit der erfindungsgemäßen, Dihydroxyaceton und Alloxan enthaltenden Zusammensetzung behandelte Haarhälfte wies eine glänzende mittelblonde Färbung auf, während die mit der Zusammensetzung nach dem Stand der Technik behandelte Haarhälfte eine deutlich blässere Hellblondfärbung zeigte.

### 3.

| | 23 | 24 | 25 | 26 | 27 | 28 | 29 | 30 | 31 | 32 | 33 |
|---|---|---|---|---|---|---|---|---|---|---|---|
| Hydroxyethyl-4,5-diaminopyrazolsulfat | 0,54 | 0,54 | 0,54 | 0,27 | | | | | 0,54 | | 0,54 |
| 3,4-Diamino-5-hydroxypyrazolsulfat | | | | | 0,48 | 0,24 | | | | | |
| 3,5-Diaminopyrazol-1-carboxamid | | | | | | | 0,31 | | | | |
| 3-Amino-5-hydroxypyrazol | | | | | | | | 0,22 | | | |
| 1-Phenyl-3-methylpyrazol-5-on | | | | | | | | | 0,39 | | |
| 3,5-Diamino-1,2,4-triazol | | | | | | | | | | 0,22 | |
| p-Toluylendiaminsulfat | | | | | | 0,50 | | 0,49 | | | |
| 4-Amino-2-hydroxytoluol | | 0,27 | | 0,13 | 0,27 | | | | | 0,27 | |
| 2-Amino-4-hydroxyethyl-aminoanisolsulfat | | | 0,62 | | | | 0,62 | | | | |
| 4-Amino-3-methylphenol | | | | 0,27 | | | | | | | |
| 1-Naphthol | | | | | | 0,16 | | | | | |
| Resorcin | | | | | | | | | | | 0,30 |
| Dihydroxyaceton | | | 0,50 | | | 0,40 | | 0,25 | 0,25 | | |
| Alloxan | | 0,50 | | | 0,40 | | 0,25 | 0,25 | | | 0,50 |
| Methylglyoxal | 0,50 | | | 0,45 | | | 0,25 | | 0,25 | 0,50 | |
| Kaliumjodid | 0,002 | 0,002 | 0,002 | 0,003 | 0,005 | 0,005 | | | | | |

| **Färbeergebnisse:** | **Ohne zusätzliche Wirkstoffe** | **Mit Wirkstoffkombination** |
|---|---|---|
| 23 | Hellmagenta | Intensives Dunkelmagenta |
| 24 | Rotkupfer | Intensives Rotkupfer |
| 25 | Violett | Tiefviolett |
| 26 | Rotkupfer | Intensives Rotkupfer |
| 27 | Rosaweiß | Orangeweiß |
| 28 | Mattviolett | Intensives Violett |
| 29 | Gelbgrau | Braungrau |
| 30 | Hellblond | Intensives Mittelblond |
| 31 | Kupferblond | Rotkupfer |
| 32 | Hellgraubraun | Graubraun |
| 33 | Hellmagenta | Dunkelmagenta |

### 3a.

In die beschriebene Grundlage wurde die folgende Oxidationsfarbstoffmischung jeweils mit und ohne Wirkstoff eingebracht und der pH-Wert so eingestellt, daß beim Vermischen mit 2%-iger wäßriger H₂O₂-Lösung im Gewichtsverhältnis 1:1 ein pH-Wert der applikationsfertigen Mischung von 6,8 erreicht wurde.
Die Mischung wurde wiederum jeweils auf Woll-Läppchen und Strähnen aus gebleichtem Menschenhaar aufgebracht, nach 15-minütiger Einwirkung ausgewaschen und getrocknet und die Färbung bewertet.

Es wurde folgendes Ergebnis erzielt:

| Zusammensetzung | | |
|---|---|---|
| | Nr. 34 | Nr. 34a |
| 1-Hydroxyethyl-4,5-diaminopyrazolsulfat | 0,54 % | 0,54 % |
| 4-Amino-2-hydroxytoluol | 0,27 % | 0,27 % |
| Methylglyoxal | 0,50 % | ― |
| Kaliumjodid | 0,002 % | ― |
| Färbung | Intensives Rotkupfer | Rotkupfer |

Die erfindungsgemäße Zusammensetzung Nr. 34 erzielt eine glänzende, intensive Ausfärbung, die derjenigen der Zusammensetzung 34a deutlich überlegen war.

### 4.

| Zusammensetzung Nr. | 35 | 36 | 37 | 38 | 39 | 40 | 41 |
|---|---|---|---|---|---|---|---|
| 3-Amino-2-(β-hydroxyethylamino)-6-methoxypyridin HCI | 0,46 | | | | | | 0,46 |
| 3-Amino-2-methylamino-6-methoxypyridin | | 0,50 | | 0,50 | | | |
| 2-Dimethylamino-5-aminopyridin | | | | | 0,47 | | |
| 2,5-Diaminopyridin | | | 0,41 | | | | |
| 2-Dimethylaminoethyl-3-hydroxypyridin | | | | | | 0,34 | |
| 2,3-Diaminopyridin | | | | | | 0,25 | |
| 2-Amino-3-hydroxypyridin | | | 0,25 | | | | |
| 2,6-Diaminopyridin | | 0,25 | | | | | |
| 2-Amino-4,6-dimethylpyridin | | | | | 0,27 | | |
| 4-Amino-2-hydroxytoluol | 0,13 | | | | | | |
| 2-Amino-4-hydroxyethylaminoanisolsulfat | 0,31 | | | | | | |
| 1-Naphthol | | | | 0,32 | | | |
| Resorcin | | | | | | | 0,25 |
| Dihydroxyaceton | | | 0,50 | 0,20 | 0,20 | | |
| Alloxan | | 0,50 | | | 0,30 | 0,50 | |
| Methylglyoxal | 0,50 | | | 0,30 | | | 0,50 |
| Kaliumjodid | 0,002 | 0,002 | 0,002 | | | | |

| **Färbeergebnisse:** | **Ohne zusätzliche Wirkstoffe** | **Mit Wirkstoffkombination** |
|---|---|---|
| 35 | Graublau | Intensives Graublau |
| 36 | Türkis | Intensives Dunkeltürkis |
| 37 | Helles Braunorange | Intensives Rotgold |
| 38 | Graugrün | Intensives Dunkelgraugrün |
| 39 | Blaßrot | Intensives Rot |
| 40 | Blaßgelb | Intensives Beigegelb |
| 41 | Gelbgrau | Kräftiges Grau |

### 4a.

In die beschriebene Grundlage wurden die folgenden Oxidationsfarbstoffmischungen jeweils mit und ohne Wirkstoff eingebracht und der pH-Wert so eingestellt, daß beim Vermischen mit 2%-iger wäßriger H₂O₂-Lösung im Gewichtsverhältnis 1:1 ein pH-Wert der applikationsfertigen Mischung von 6,8 erreicht wurde.
Die Mischungen wurden wiederum jeweils auf Woll-Läppchen und Strähnen aus gebleichtem Menschenhaar aufgebracht, nach 15-minütiger Einwirkung ausgewaschen und getrocknet und die Färbung bewertet.
Es wurde folgendes Ergebnis erzielt:

| Nr. | 42 | 42a | 43 | 43a |
|---|---|---|---|---|
| 3-Amino-2-methylamino-6-methoxypyridin | 0,50 | 0,50 | 0,75 | 0,75 |
| 2-Amino-3-hydroxypyridin | 0,25 | 0,254 | ― | 0,25 |
| 1-Naphthol | ― | ― | 0,48 | 0,48 |
| Alloxan | 0,50 | ― | ― | ― |
| Methylglyoxal | ― | ― | 0,50 | |

Die erfindungsgemäße Zusammensetzung Nr. 42 erzielte eine intensive braunorange Ausfärbung, während mit der Rezeptur Nr. 42a lediglich eine blaßorange Färbung erreicht wurde.
Die erfindungsgemäße Zusammensetzung Nr. 43 erzielte eine intensive graubraune Ausfärbung, während mit der Rezeptur Nr. 43a lediglich eine schwach graubraune Ausfärbung erreicht wurde.

## Patentansprüche

1. Mittel zum Färben von menschlichen Haaren, enthaltend mindestens ein Oxidationsfarbstoffvorprodukt, ausgewählt aus 4-Aminophenol und dessen Derivaten der allgemeinen Formel (I) 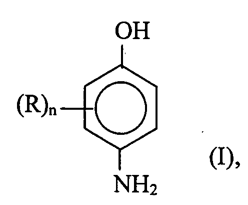 worin R eine C₁-C₃-Alkylgruppe, eine Hydroxy-C₁-C₃-alkylgruppe oder ein Halogenatom, insbesondere Cl, und n eine Zahl von 0 bis 2 bedeuten, oder 2-Aminophenol in wäßriger Grundlage, **gekennzeichnet durch** einen Gehalt an 0,0001 bis 5 Gew.-% Dihydroxyaceton und/oder Alloxan und/oder Methylglyoxal und/oder Kaliumjodid, berechnet auf die Gesamt-zusammensetzung.

2. Mittel nach Anspruch 1, enthaltend mindestens ein Oxidationsfarbstoffvorprodukt, ausgewählt aus 4-Aminophenol, 4-Amino-3-methylphenol, 2-Chlor-4-aminophenol, 2,6-Dichlor-4-aminophenol, 2,4-Diaminophenol, 2,6-Dibrom-4-aminophenol und/oder 2-Aminophenol.

3. Mittel zum Färben von menschlichen Haaren, enthaltend mindestens ein Oxidationsfarbstoffvorprodukt, ausgewählt aus 1,4-Diaminobenzol und substituierten p-Phenylendiaminen bzw. deren wasserlöslichen Salzen in wäßriger Grundlage, **gekennzeichnet durch** einen Gehalt an 0,0001 bis 5 Gew.-% Dihydroxyaceton und/oder Alloxan und/oder Methylglyoxal und/oder Kaliumjodid, berechnet auf die Gesamtzusammensetzung des Mittels.

4. Mittel nach Anspruch 3, enthaltend als substituierte p-Phenylendiamine 2,5-Diaminotoluol, 2-n-Propyl- bzw. 2-Ethyl-p-phenylendiamin, 2,6-Dimethyl-pphenylendiamin, 2-(2,5-Diaminophenyl)ethanol, 1-Amino-4-bis-(2'-hydroxyethyl)-aminobenzol, 2-(2-Hydroxyethylamino)-5-aminotoluol, 4,4'-Diaminodiphenylamin, 4-Aminodiphenylamin, 2-Amino-5-N,N-diethylaminotoluol, 4-Amino-N-ethyl-N-isopropylanilin, 2-Chlor-p-phenylendiamin, 1-β-Hydroxyethyl-2,5-diamino-4-chlorbenzol, 1-β-Hydroxyethyl-2,5-diamino-4-methylbenzol, 2-Methoxy-p-phenylendiamin, N,N-Diethyl-p-phenylendiamin, 1-Amino-4-β-methoxyethylaminobenzol, 1-Dimethylamino-4-aminobenzol, 1-Hydroxy-2,5-diamino-4-methylbenzol, 1-Hydroxymethyl-2,5-diaminobenzol, 1,3-Dimethyl-2,5-diaminobenzol, 1,4-Diaminoisopropylbenzol und/oder 1-Amino-4-β-hydroxypropylaminobenzol bzw.

5. Mittel zum Färben von menschlichen Haaren, enthaltend mindestens ein Oxidationsfarbstoffvorprodukt, ausgewählt aus Pyrazol und seinen Derivaten bzw. deren wasserlöslichen Salzen und/oder einem substituierten Triazol in wäßriger Grundlage, **gekennzeichnet durch** einen Gehalt an 0,0001 bis 5 Gew.-% Dihydroxyaceton und/oder Alloxan und/oder Methylglyoxal und/oder Kaliumjodid, berechnet auf die Gesamtzusammensetzung des Mittels.

6. Mittel nach Anspruch 5, enthaltend als Pyrazol- bzw. Triazolderivat mindestens eine Verbindung ausgewählt aus 1-Hydroxyethyl-4,5-diaminopyrazol, 3,4-Diamino-5-hydroxypyrazol, 3,5-Diaminopyrazol, 3,5-Diaminopyrazol-1-carboxamid, 3-Amino-5-hydroxypyrazol, 1-Phenyl-2-methylpyrazol, 1-Phenyl-3-methylpyrazol-5-on, 3,5-Dimethylpyrazol, 3,5-Dimethylpyrazol-1-methanol und/oder 3,5-Diamino-1,2,4-triazol bzw. deren wasserlösliche Salze.

7. Mittel zum Färben von menschlichen Haaren, enthaltend mindestens ein Oxidationsfarbstoffvorprodukt, ausgewählt aus Aminopyridinen und deren wasserlöslichen Salzen in wäßriger Grundlage, **gekennzeichnet durch** einen Gehalt an 0,0001 bis 5 Gew.-% Dihydroxyaceton und/oder Alloxan und/oder Methylglyoxal und/oder Kaliumjodid, berechnet auf die Gesamtzusammen-setzung des Mittels.

8. Mittel nach Anspruch 7, enthaltend als Aminopyridin 2,5-Diaminopyridin, 2,3-Diaminopyridin, 2,6-Diaminopyridin, 3-Amino-2-methylamino-6-methoxypyridin, 2-Dimethyl-5-aminopyridin, 2-Dimethylaminoethyl-3-hydroxypyridin, 2-Amino-4,6-dimethylpyridin, 2-Amino-3-hydroxypyridin, 3-Amino-2-(β-hydroxyethylamino)-6-methoxypyridin, 2,6-Dimethylamino-5-aminopyridin, 2-Di-(hydroxyethyl)amino-5-aminopyridin, 2-Hydroxyethylamino-5-aminopyridin und/oder dessen wasserlösliche Salze.

9. Mittel nach Anspruch 1 und/oder 2, 3, 4, 5, 6, 7, **dadurch gekennzeichnet, daß** es zusätzlich mindestens eine Kupplersubstanz enthält.

10. Mittel nach Anspruch 9, enthaltend mindestens eine Kupplersubstanz, ausgewählt aus Resorcin, 2-Methylresorcin, 4-Chlorresorcin, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihydroxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1,3-Diaminobenzol, 1-Amino-3-(2'hydroxyethylamino)benzol, 1-Amino-3-[bis(2'hydroxyethyl)amino]benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 4-Hydroxy-1,2-methylendioxybenzol, 2,4-Diamino-3-chlorphenol, 5-Amino-2-methoxyphenol und/oder 1-Methoxy-2-amino-4-(2'-hy-droxyethylamino)benzol bzw. deren wasserlöslichen Salzen.

11. Mittel nach einem oder mehreren der Ansprüche 1 bis 10, enthaltend 0,001 bis 1 Gew.-% Dihydroxyaceton und/oder Alloxan und/oder Methylglyoxal und/oder Kaliumjodid, bezogen auf die Gesamtzusammensetzung.

12. Verwendung von Dihydroxyaceton und/oder Alloxan und/oder Methylglyoxal und/oder Kaliumjodid in Haarfärbemitteln auf Basis mindestens eines Oxidationsfarbstoffvorprodukts, ausgewählt aus 4-Aminophenol, 4-Amino-3-methylphenol, 2-Chlor-4-aminophenol, 2,6-Dichlor-4-aminophenol, 2,4-Diaminophenol, 2,6-Dibrom-4-aminophenol, 2-Aminophenol und/oder 1,4-Diaminobenzol und substituierten p-Phenylendiaminen und/oder Pyrazol und seinen Derivaten und/oder substituierten Triazolen und/oder Aminopyridinen bzw. deren wasserlöslichen Salzen.
